# EUROPÄISCHE PATENTSCHRIFT

(11) **EP 0 353 189 B1**
(45) Veröffentlichungstag und Bekanntmachung des Hinweises auf die Patenterteilung: **12.01.1994**
(21) Anmeldenummer: 89810546.5
(22) Anmeldetag: 19.07.1989
(51) Int. Cl.: C07D 453/02, G01N 31/22

(54) **Kupfer- und Nickeldihalogenidkomplexe, Verfahren zu deren Herstellung und deren Verwendung**
Copper and nickel dihalogenide complexes, process for their preparation and their use
Complexes de dihalogénures du cuivre et du nickel, leur procédé de préparation et leur utilisation

(30) Priorität: 28.07.1988 CH 2868/88
(43) Veröffentlichungstag der Anmeldung: 31.01.1990
(73) Patentinhaber: CIBA-GEIGY AG, 4002 Basel (CH)
(72) Erfinder: Schmidhalter, Beat, Dr., CH-6208 Oberkirch (CH)

(56) Entgegenhaltungen:
- US-A- 4 567 019
- THE JOURNAL OF ORGANIC CHEMISTRY, Band 35, Nr. 2, Seiten 503-505, Februar 1970; D.L. COFFEN et al.: "Isomeric transition metal complexes of trans-2-(2'-quinolyl)-methylene-3-quinuclidinones"
- JOURNAL OF THE CHEMICAL SOCIETY, DALTON TRANSACTIONS, Seiten 762-765, 1975; G.J. LONG et al.: "The transition-metal chemistry of quinuclidinone-containing ligands. Part VI. A study of the thermal properties of several complexes with trans-2-(2-quinolyl)methylenequinuclidin-3-one 1"
- COORDINATION CHEMISTRY REVIEWS, Band 47, Nr. 1-2, Seiten 125-133, November 1982; D.R. BLOOMQUIST et al.: "Thermochromic phase transitions in transition metal salts"
- AUSTRALIAN JOURNAL OF CHEMISTRY, Band 26, Nr. 5, Seiten 1809-1814, Mai 1973; E.J.BROWNE: "Azole aldehyde condensations"

## Beschreibung

Die Erfindung betrifft Kupfer(II)- und Nickel(II)-dichlorid- und -dibromid-Komplexe mit mindestens in 6-Stellung substituierten 2-(2'-Pyridylmethylen)-chinuclidin-3-onen, diese Chinuclidin-3-one, ein Verfahren zur Herstellung der Komplexe und deren Verwendung als kryochrome und thermochrome Warnindikatoren.

Thermochrome Materialien werden zur optischen Anzeige von Temperaturänderungen verwendet, die zum Beispiel Funktionsstörungen in Bauelementen auslösen können. In der DE-OS 2 951 921 sind für diesen Zweck eine Anzahl organischer Verbindungen beschrieben, deren Farbänderung auf einer Zersetzung beruht. In der US-PS 4,567,019 werden für diese Verwendung reversible binäre Systeme aus Bis(p-aminophenyl)-phthaliden und organischen Säuren vorgeschlagen.

D.R. Bloomquist et al. beschreiben in Coord. Chem. Rev., 47, S. 125-133 (1982) Dichloro[2-(2'-chinonyl)-methylen-chinuclidin-3-on]nickel(II), dessen gelbe Modifikation bei 230°C in eine violette Modifikation übergeht. Die ursprüngliche gelbe Farbe kann nur durch Abkühlen auf etwa -78°C wieder erhalten werden. Dieser Nickel-Komplex weist somit eine ausgeprägte thermische Hysterese auf, so dass es sich nicht als reversibles System eignet. Es wird ferner erwähnt, dass der entsprechende Nickeldibromidkomplex und ein in 6'-Stellung mit Methoxy substituiertes Derivat des Nickeldichloridkomplexes keinen Thermochromismus besitzen.

Es wurde gefunden, dass Kupfer(II)- und Nickel(II)-dichlorid- oder -dibromid-komplexe sogar zwei mit einer intensiven Farbänderung verbundene Umwandlungstemperaturen aufweisen, wenn sie als Komplexligand ein in 6'-Stellung substituiertes 2-(2'-Pyridyl)-methylen-chinuclidin-3-on enthalten.

Ein Gegenstand der Erfindung sind Verbindungen der Formel I 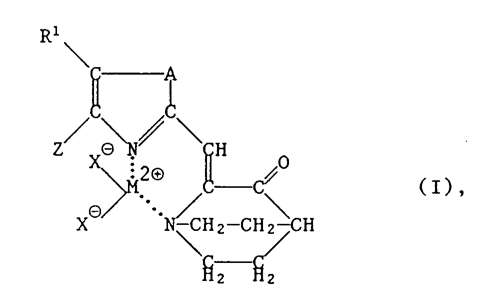
worin
X^{⊖} für Cl^{⊖} oder Br^{⊖} steht,
M Cu^{2⊕} oder Ni^{2⊕} bedeutet,
Z ein Halogenatom, -NH₂, C₁-C₄-Alkyl oder C₁-C₄-Alkoxy darstellt,
R¹ für H, Methyl, Methoxy, Halogen oder -NH₂ steht, und A die Gruppe 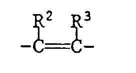
bedeutet, worin R² und R³ unabhängig die gleiche Bedeutung wie R¹ haben.
X^{⊖} steht insbesondere für Cl^{⊖}.

Bei der Gruppe Z in der Bedeutung von Halogen handelt es bevorzugt um F, Cl und Br. Z in der Bedeutung von Alkyl und Alkoxy kann z.B. Methyl, Ethyl, n- und i-Propyl, n-, i- und t-Butyl, Methoxy, Ethoxy, n- und i-Propoxy, n-, i- und t-Butoxy sein. Besonders bevorzugt steht Z für Methyl.

R¹ kann in der Bedeutung von Halogen, F, Cl und Br sein. Insbesondere bedeutet R¹ H.

A steht für die Gruppe 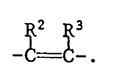
R² und R³ haben unabhängig die gleiche Bedeutung wie R¹. Insbesondere bedeuten R² und R³ H.

In einer bevorzugten Ausführungsform handelt es sich bei der Verbindung der Formel I um den Kupfer(II)-dichloridkomplex mit 2-[(6′-Methyl-2′-pyridyl)-methylen]-chinuclidin-3-on.

In einer anderen bevorzugten Ausführungsform handelt es sich bei der Verbindung der Formel I um den Nickel(II)-dichloridkomplex mit 2-[(6′-Methyl-2′-pyridyl)-methylen]-chinuclidin-3-on.

Die erfindungsgemässen Verbindungen können unterschiedliche Mengen Kristallwasser enthalten.

Ein weiterer Gegenstand der Erfindung ist ein Verfahren zur Herstellung von Verbindungen der Formel I gemäss Anspruch 1, dadurch gekennzeichnet, dass man ein Kupfer(II)-dichlorid oder -dibromid oder ein Nickel(II)-dichlorid oder -dibromid mit einer Verbindung der Formel II 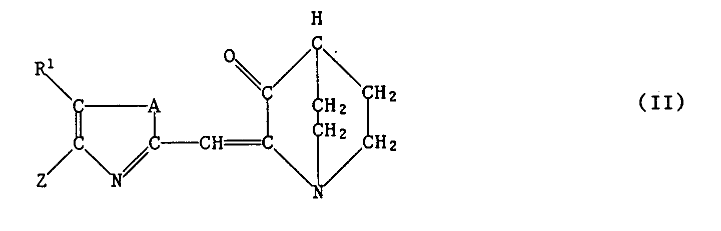
umsetzt, wobei R¹, A und Z die in Anspruch 1 angegebene Bedeutung haben.

Die Umsetzung wird bevorzugt in alkoholischer oder wässrig-alkoholischer Lösung durchgeführt. Geeignete Alkohole sind z.B. Methanol, Ethanol, n-oder i-Propanol, n-, i- oder t-Butanol, Pentanole und Hexanole.

Die Reaktionstemperatur kann z.B. 50 bis 250°C, vorteilhaft 80 bis 200°C betragen.

Die kristallinen Verbindungen der Formel I fallen beim Abkühlen des Reaktionsgemisches aus. Sie können durch Filtrieren isoliert und mit üblichen Methoden, wie z.B. Auswaschen mit einem Nichtlösungsmittel oder Umkristallisation, gereinigt werden.

Die Verbindungen der Formel II sind in an sich bekannter Weise durch Kondensation von mindestens in 6-Stellung substituierten Pyridin-2-aldehyden der Formel III 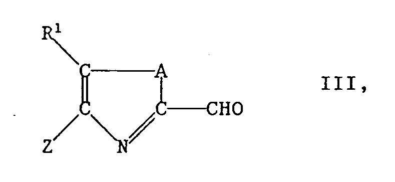
worin A, Z und R¹ die zuvor angegebenen Bedeutungen haben, mit Chinuclidin-3-on oder dessen Salzen, wie z.B. den Hydrochloriden, erhältlich. Die Umsetzung wird vorteilhaft in Gegenwart eines Alkalimetallalkoholates durchgeführt. Geeignete Alkalimetalle sind z.B. Li, Na und K. Die Verbindungen der Formel III sind bekannt oder nach bekannten Verfahren herstellbar.

Ein weiterer Gegenstand der Erfindung sind Verbindungen der Formel II 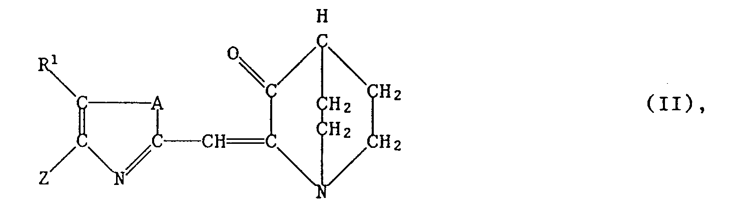
worin
Z ein Halogenatom, -NH₂, C₁-C₄-Alkyl oder C₁-C₄-Alkoxy darstellt,
R¹ für H, Methyl, Methoxy, Halogen oder -NH₂ steht, und A die Gruppe 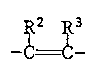
bedeutet, worin R² und R³ unabhängig die gleiche Bedeutung wie R¹ haben. Für A, Z, R¹, R² und R³ gelten die gleichen Bevorzugungen wie für die Verbindungen der Formel I. Besonders bevorzugt ist 2-(6'-Methyl-2'-pyridyl)-methylenchinuclidin-3-on.

Die Verbindungen der Formel I sind gefärbte kristalline Verbindungen, die überraschend zwei mit einer Farbänderung verbundene Umwandlungstemperaturen aufweisen, die reversibel oder irreversibel sein können. Die Farbumwandlung kann sogar bei tiefen Temperaturen stattfinden, z.B. bei Temperaturen um -190°C. Hierdurch werden auch Tieftemperaturanwendungen erschlossen, z.B. für den Bereich der Supraleiter.

Die Verbindungen der Formel I eignen sich als Warn- und Temperaturindikatoren, z.B. zur Feststellung bzw. Verhinderung von Funktionsstörungen bei Bauelementen auf Grund von Temperaturänderungen oder -schwankungen (siehe z.B. US-PS 4,567,019 und DE-OS 2 951 921). Die Verbindungen können hierzu direkt auf einen Gegenstand aufgebracht und gegebenenfalls mit einer Schutzschicht versehen werden. Sie können hierzu auch einem Bindemittel z.B. aus Kunststoffen, oder Kunststoffbauteilen einverleibt werden.

Ein weiterer Gegenstand der Erfindung ist die Verwendung von Verbindungen der Formel I als kryochrome oder thermochrome Warnindikatoren oder Temperaturindikatoren.

Die nachfolgenden Beispiele erläutern die Erfindung näher.

### A) Herstellung von Ausgangsverbindungen

### 2-(6′-Methyl-2′-pyridyl)-methylenchinuclidin-3-on

Zu einer gerührten Lösung von 1,74 g (0,075 mol) Natrium in 50 ml absolutem Ethanol wird im Verlauf von 5 min ein Gemisch aus 6,24 g (0,05 mol) 6-Methyl-pyridin-2-carbaldehyd und 8,08 g (0,05 mol) 3-Chinuclidonhydrochlorid in 125 ml Ethanol gegeben. Unter Rühren wird die gelbe Suspension langsam zum Sieden erhitzt und unter Rückfluss 30 min erhitzt. Nach beendeter Reaktion wird auf Raumtemperatur abgekühlt und das überschüssige Na-Ethanolat mit H₂O zersetzt. Die filtrierte Lösung wird auf die Hälfte ihres Volumens eingeengt und zur Kristallisation stehen gelassen. Es werden 10,2 g (89,5 % d.Th.) hellgelbe Kristalle vom Smp. 108-109°C erhalten.
Elementaranalyse: 73,43 % C, 7,14 % H, 12,32 % N, 7,06 % O (berechnet: 73,66 % C, 7,07 % H, 12,27 % N, 7,0 % O).

### B) Herstellungsbeispiele

### Beispiel 1: Cu(II)dichlorid-Komplex mit 2-(6′-methyl-2′-pyridyl)-methylenchinuclidin-3-on

1,14 g (0,05 mol) 2-(6′-Methyl-2′-pyridyl)-methylenchinuclidin-3-on wird in 100 ml n-Butanol gelöst und auf Rückflusstemperatur erhitzt. Danach werden 0,28 g (0,05 mol) CuCl₂·2H₂O in 12,5 ml 95 % Ethanol gelöst und zum Rückfluss erhitzt. Die erhaltene Lösung wird innert 5 min in die erste Lösung eingebracht. Nach beendeter Zugabe wird auf Raumtemperatur abgekühlt und der ausgefallene orangefarbene Feststoff filtriert, gut mit Hexan gewaschen und im Vakuum bei 80°C getrocknet.
Es werden 1,66 g (92,7 % d.Th.) orange Kristalle erhalten.
Elementaranalyse: 46,30 % C, 4,52 % H, 7,87 % N, 19,57 % Cl, 17,5 % Cu (berechnet: 46,36 % C, 4,45 % H, 7,72 % N, 19,55 % Cl, 17,52 % Cu, 4,41 % O).

Das Produkt wird aus absolutem Ethanol umkristallisiert. Hierbei lässt sich eine polymorphe hellgrüne Kristallform isolieren. Die unterschiedlichen Kristallmodifikationen werden durch Röntgenbeugungsdiagramme nachgewiesen. Aus der hellgrünen Form kann nach Lösen in n-Butanol die orange Form rekristallisiert werden.

### Beispiel 2: Ni(II)dichlorid-Komplex mit 2-(6′-methyl-2′-pyridyl)-methylenchinuclidin-3-on

Zu einer gerührten Mischung aus 2,28 g (0,01 mol) 2-(6′-Methyl-2′-pyridyl)-methylenchinuclidin-3-on in 200 ml n-Butanol wird bei Rückflusstemperatur eine heisse Lösung von 2,28 g (0,01 mol) Nickelchlorid 6·H₂O in 25 ml Ethanol gegeben. Nach dem Abkühlen wird das Lösungsmittel im Vakuum entfernt bis zur Ausfällung eines hellgrünen Ni(II)-Komplexes. Das gesammelte feste Produkt wird gründlich mit Hexan gewaschen und dann im Vakuum bei 50°C getrocknet.
Ausbeute: 3,58 g (91,9 % d.Th.) des hellgrünen Komplexes.
Elementaranalyse: 44,67 % C, 4,92 % H, 7,55 % N, 18,80 % Cl, 15,8 % Ni (berechnet: 44,49 % C, 4,87 % H, 7,41 % N, 18,76 % Cl, 15,54 % Ni, 8,93 % O). Die Verbindung enthält 5,3 % Kristallwasser.

### Beispiel 3: Ni(II)dibromid-Komplex mit 2-(6′-Chlor-2′-pyridyl)-methylenchinuclidin-3-on

a) 6-Chlor-pyridin-2-carbaldehyd wird gemäss Vorschrift J. chem. Commun. S. 410-411 (1974) hergestellt:
   2,5 g (0,108 Mol) Natrium werden unter Argon in 70 ml absolutem Ethanol vorgelegt und unter Rühren gelöst. Eine Suspension von 11,7 g (0,071) 3-Chinuclidonhydrochlorid in 80 ml absolutem Ethanol und 10 g (0,071) 6-Chlor-pyridin-2-carbaldehyd in 100 ml absolutem Ethanol wird zugesetzt und die Reaktionsmischung während 30 Min. am Rückfluss erhitzt. Dann lässt man auf Raumtemperatur abkühlen und das ausgefallene Produkt wird abfiltriert, mit Wasser gewaschen und getrocknet.
   Man erhält 1,72 g (89,6 % d. Th.) gelblich kristalline Substanz, die bei 135°C schmilzt.
b) Zu einer gerührten Mischung aus 1,24 g (0,05 mol) 2-(6′-Chlor-2′-pyridyl)-methylenchinuclidin-3-on in 100 ml n-Butanol wird bei Rückflusstemperatur eine heisse Lösung von 1,11 g (0,05 mol) NiBr₂ in 12,5 ml Ethanol gegeben. Nach dem Abkühlen wird das Lösungsmittel auf die Hälfte eingeengt und das kristalline Produkt durch Filtration gesammelt. Der Festkörper wird in n-Hexan gewaschen und im Vakuum bei 70°C getrocknet. Man erhält 2,14 g (91,5 % d.Th.) einer gelblichen kristallinen Substanz.
   Elementaranalyse: 33,5 % C, 2,93 % H, 6 % N, 7,5 % Cl, 33,38 % Br, 12,5 % Ni,
   (berechnet: 33,42 % C, 2,80 % H, 6,00 % N, 7,59 % Cl, 34,20 % Br, 12,57 % Ni, 3,42 % O). Die Verbindung enthält 1,11 % Kristallwasser.

### C) Anwendungsbeispiele

Beispiel 4: Die orange Form des Cu-Komplexes von Beispiel 1 wird auf einen Glasträger aufgebracht und gekühlt. Bei -196°C erfolgt ein Farbwechsel nach intensiv gelb, der reversibel ist. Beim Erwärmen erfolgt bei 193°C ein Farbwechsel nach dunkelgrün, der irreversibel ist.

Beispiel 5: Der hellgrüne Ni-Komplex wird analog Beispiel 4 erwärmt. Bei 119°C tritt ein Farbwechsel nach intensiv gelb auf, der irreversibel ist. Bei 160°C tritt ein Farbwechsel nach violett auf, der irreversibel ist.

Beispiel 6: Der hellgelbe Ni-Komplex wird analog Beispiel 4 erwärmt. Bei 140°C tritt ein Farbwechsel nach dunkel gelb auf, der reversibel ist. Bei -196°C erfolgt ein Farbwechsel nach goldgelb, der reversibel ist.

## Patentansprüche

1. Verbindungen der Formel I 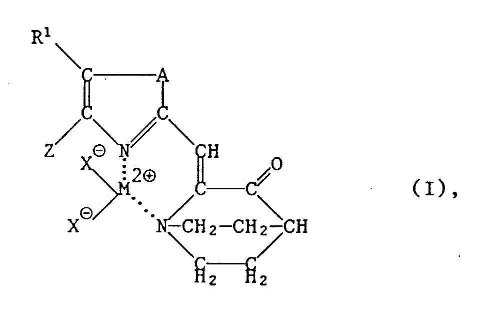 worin
X^{⊖} für Cl^{⊖} oder Br^{⊖} steht,
M Cu^{2⊕} oder Ni^{2⊕} bedeutet,
Z ein Halogenatom, -NH₂, C₁-C₄-Alkyl oder C₁-C₄-Alkoxy darstellt,
R¹ für H, Methyl, Methoxy, Halogen oder -NH₂ steht, und A die Gruppe 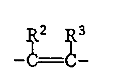 bedeutet, worin R² und R³ unabhängig die gleiche Bedeutung wie R¹ haben.

2. Verbindungen der Formel I gemäss Anspruch 1, worin X^{⊖} für Cl^{⊖} steht.

3. Verbindungen der Formel I gemäss Anspruch 1, worin Z Methyl bedeutet.

4. Verbindungen der Formel I gemäss Anspruch 1, worin R¹ für H steht.

5. Verbindungen der Formel I gemäss Anspruch 1, worin R² und R³ je H bedeuten.

6. Verbindungen der Formel I gemäss Anspruch 1, dadurch gekennzeichnet, dass es sich um den Kupfer(II)-dichloridkomplex mit 2-[(6'-Methyl-2'-pyridyl)-methylen]-chinuclidin-3-on handelt.

7. Verbindungen der Formel I gemäss Anspruch 1, dadurch gekennzeichnet, dass es sich um den Nickel(II)-dichloridkomplex mit 2-[(6'-Methyl-2'-pyridyl)-methylen]-chinuclidin-3-on handelt.

8. Verfahren zur Herstellung von Verbindungen der Formel I gemäss Anspruch 1, dadurch gekennzeichnet, dass man ein Kupfer(II)-dichlorid oder -dibromid oder ein Nickel(II)-dichlorid oder -dibromid mit einer Verbindung der Formel II 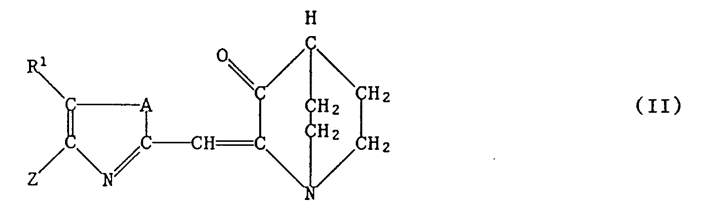 umsetzt, wobei R¹, A und Z die in Anspruch 1 angegebene Bedeutung haben.

9. Verfahren gemäss Anspruch 8, dadurch gekennzeichnet, dass man die Umsetzung in alkoholischer oder wässrig-alkoholischer Lösung durchführt.

10. Verbindungen der Formel II 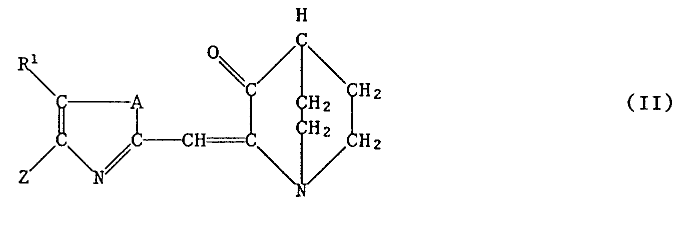 worin
Z ein Halogenatom, -NH₂, C₁-C₄-Alkyl oder C₁-C₄-Alkoxy darstellt,
R¹ für H, Methyl, Methoxy, Halogen oder -NH₂ steht, und A die Gruppe 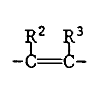 bedeutet, worin R² und R³ unabhängig die gleiche Bedeutung wie R¹ haben.

11. Verwendung von Verbindungen der Formel I gemäss Anspruch 1 als kryochrome oder thermochrome Warnindikatoren oder Temperaturindikatoren.

## Claims

1. A compound of formula I 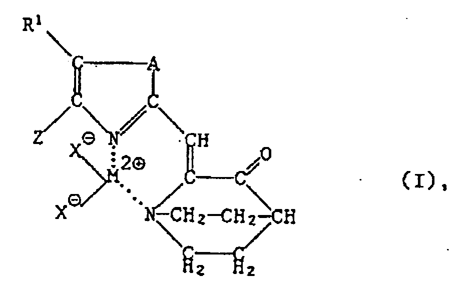 in which
X^{⊖} is Cl^{⊖} or Br^{⊖},
M is Cu^{2⊕} or Ni^{2⊕},
Z is a halogen atom, -NH₂, C₁-C₄alkyl or C₁-C₄alkoxy,
R¹ is hydrogen, methyl, methoxy, halogen or -NH₂, and
A is the group 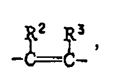 in which R² and R³ each independently have the same meaning as R¹.

2. A compound of formula I according to claim 1, in which X^{⊖} is Cl^{⊖}.

3. A compound of formula I according to claim 1, in which Z is methyl.

4. A compound of formula I according to claim 1, in which R¹ is hydrogen.

5. A compound of formula I according to claim 1, in which R² and R³ are each hydrogen.

6. A compound of formula I according to claim 1, which is the copper(II) dichloride complex of 2-[(6'-methyl-2'-pyridyl)methylene]-3-quinuclidinone.

7. A compound of formula I according to claim 1, which is the nickel(II) dichloride complex of 2-[(6'-methyl-2'-pyridyl)methylene]-3-quinuclidinone.

8. A process for the preparation of a compound of formula I according to claim 1, which comprises reacting a copper(II) dichloride or dibromide or a nickel(II) dichloride or dibromide with a compound of formula II 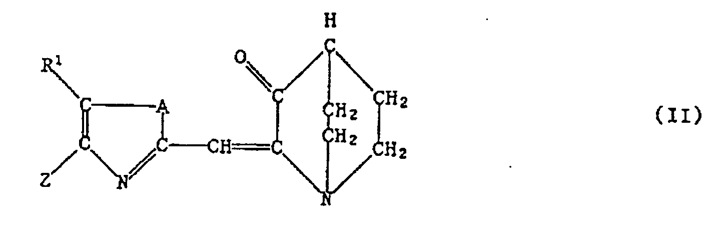 where R¹, A and Z are as defined in claim 1.

9. A process according to claim 8, wherein the reaction is carried out in alcoholic or aqueous-alcoholic solution.

10. A compound of formula II 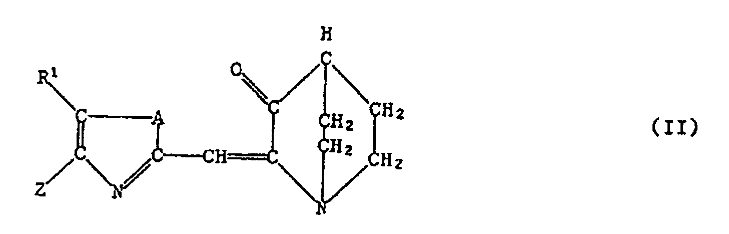 in which
Z is a halogen atom, -NH₂, C₁-C₄alkyl or C₁-C₄alkoxy,
R¹ is hydrogen, methyl, methoxy, halogen or -NH₂, and
A is the group 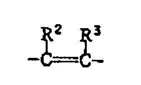 in which R² and R³ each independently have the same meaning as R¹.

11. Use of a compound of formula I according to claim 1 as cryochromic or thermochromic warning indicators or temperature indicators.

## Revendications

1. Composés de formule I 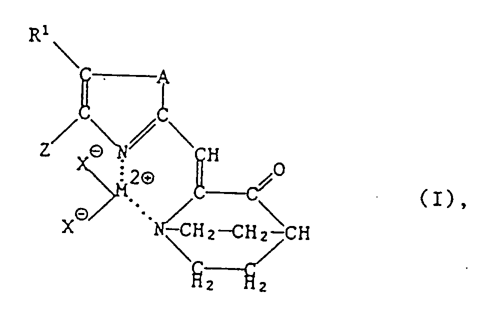 dans laquelle ,
X⁻ représente Cl⁻ ou Br⁻,
M représente Cu ^{2⊕} ou Ni^{2⊕},
Z représente un atome d'halogène, un groupe -NH₂, alkyle en C₁ à C₄ ou alcoxy en C₁ à C₄,
R¹ représente H, un groupe méthyle, méthoxy, halogéno ou -NH₂; et A représente le groupe : 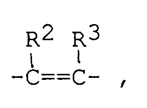 dans lequel R₂ et R³ ont, indépendamment, chacun le même sens que R¹.

2. Composés de formule I selon la revendication 1, dans lesquels X⁻ représente Cl⁻.

3. Composés de formule I selon la revendication 1, dans lesquels Z représente un groupe méthyle.

4. Composés de formule I selon la revendication 1, dans lesquels R¹ représente H.

5. Composés de formule I selon la revendication 1, dans lesquels R² et R³ représentent chacun H.

6. Composés de formule I selon la revendication 1, caractérisés en ce qu'il s'agit du complexe de dichlorure de cuivre-(II) avec la 2-[(6'-méthyl-2'-pyridyl)-méthylène]-quinuclidine-3-one.

7. Composés de formule I selon la revendication 1, caractérisés en ce qu'il s'agit du complexe de dichlorure de cuivre (II) avec la 2-[(6'-méthyl-2'-pyridyl)-méthylène]-quinuclidine-3-one.

8. Procédé pour préparer des composés de formule I selon la revendication 1, caractérisé en ce qu'on fait réagir un dichlorure ou dibromure de cuivre-(II) ou un dichlorure ou dibromure de nickel(II) avec un composé de formule II : 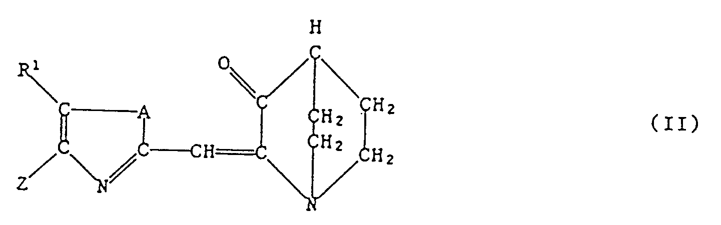 dans laquelle R¹, A et Z ont le sens indiqué à la revendication 1.

9. Procédé selon la revendication 8, caractérisé en ce qu'on conduit la réaction en solution alcoolique ou hydro-alcoolique.

10. Composés de formule II 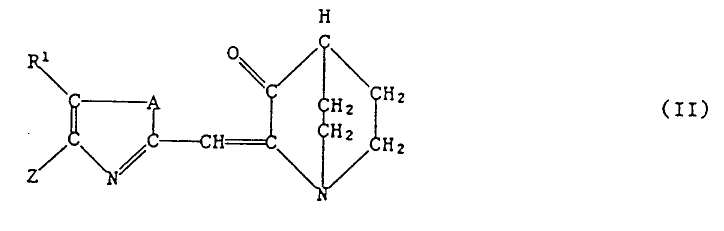 dans laquelle
Z représente un atome d'halogène, un groupe -NH₂, alkyle en C₁à C₄ ou alcoxy en C₁ à C₄ ,
R¹ représente H, un groupe méthyle, méthoxy, halogéno ou -NH₂ ; et A représente le groupe 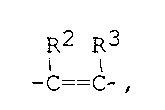 dans lequel R² et R³ ont chacun, indépendamment, le même sens que R¹.

11. Utilisation de composés de formule I selon la revendication 1, à titre d'indicateurs cryochromes ou thermochromes d'alarme ou de surveillance ou bien d'indicateurs de température.
